# EUROPEAN PATENT APPLICATION

(11) **EP 0 569 122 A1**
(43) Date of publication of application: **10.11.1993**
(21) Application number: 93302111.5
(22) Date of filing: 19.03.1993
(51) Int. Cl.: C07D 207/337, A61K 31/40, C12P 17/10

(54) **Pyrrole propionic acid amide, its production and use as calpain inhibitor**

(30) Priority: 20.04.1992 JP 99666/92; 05.02.1993 JP 19001/93
(71) Applicant: THE KITASATO INSTITUTE, Minato-ku Tokyo (JP)
(72) Inventor: Omura, Satoshi, Setagaya-ku, Tokyo (JP); Tanaka, Haruo, Machida-shi, Tokyo (JP); Shiomi, Kazuro, Room 202 Ono Building, Shibuya-ku, Tokyo (JP); Liu, Jing Rong, Fuchengmen Nandajie, Beijing (CN)
(74) Representative: Woods, Geoffrey Corlett

(57) **Abstract**

A compound of formula: 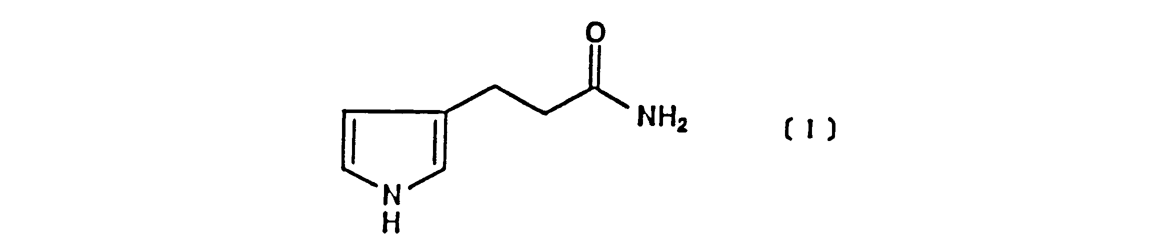
or a pharmaceutically or veterinarilly acceptable salt thereof, is useful as a calpain inhibitor.

## Description

This invention relates to a novel calpain inhibitor, cystamidin A and to its production.

Calpain is a calcium-dependent cysteine protease which occurs in various mammalian tissues. The enzyme is presumed to play a central role in physiological or pathological events, and to be involved in the activation of kinase series enzymes such as protein kinase C and phosphorylase kinase B by limiting proteolysis and in the decomposition of cytoskeletal protein and of growth-factor- and hormone-receptors. Diseases related to calpain have been known. It is strongly suggested to be involved in turnover of muscle in muscular dystrophy, for example, disappearing of Z line in skeletal muscle. (Experimental Medicine, 5 : 942 (1987).

Furthermore, in a cell infected with human T-cell leukaemia virus, extremely increased activities of calpain and interleukin 2 receptor are observed. This may be caused by irregular reaction of cells to growth factor due to alteration of receptor activity by an action of calpain on cytoskeletal protein. (Biochemistry, 57 : 1202 (1985)). Calpain is also thought to have a relation to myocardial infarction (Experimental Medicine, 5 : 937 (1987)), demyelination (Modern Medicine, 43 : 813 (1988)) and inflammation (ibid., 43 : 776 (1988)).

In a physiological condition, calpastatin, which is a specific inhibitory protein on calpain, is known and is expected to be applied as an effective therapeutic for various excessive calpain-related syndromes. Calpastatin is, however, a high molecular weight-protein and hence it is difficult to apply as a medicine. A low molecular weight-calpain inhibitor is therefore required.

We have found that a microorganism belonging to the genus Streptomyces produces a calpain inhibitor in its cultured broth.

The present invention provides a compound (cystamidin A) of the formula: 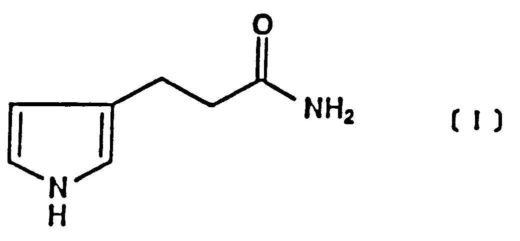
or a pharmaceutically or veterinarilly acceptable salt thereof.

The present invention also provides a process for the production of the compound of formula (I), or a pharmaceutically or veterinarilly acceptable salt thereof, which comprises culturing a microorganism belonging to the genus Streptomyces which is capable of producing the compound of formula (I) in a nutrient medium, isolating the compound of formula (I) from the cultured mass and, if desired, salifying the compound of formula (I) thus obtained.

The compound of formula (I) may be in free form or in the form of a pharmaceutically or veterinarilly acceptable salt thereof. Examples of salts are inorganic acid salts such as the hydrochloride and sulfate, and organic acid salts such as the citrate, tartrate or succinate.

The attached drawings show the following:
- Fig 1:: UV spectrum of cystamidin A;
- Fig 2:: IR spectrum of cystamidin A;
- Fig 3:: ¹H-NMR spectrum of cystamidin A; and
- Fig 4:: ¹³C-NMR spectrum of cystamidin A.

Cystamidin A of the present invention can be produced by culturing a cystamidin A producing microorganism belonging to the genus Streptomyces in a nutrient medium, accumulating the cystamidin A in a medium and isolating calpain inhibitor cystamidin A therefrom. The cystamidine A producing microorganism can be a cystamidin A producing microorganism Streptomyces and is not limited a strain illustrated in this specification. Preferable example of cystamidin A producing microorganism is Streptomyces sp. KP-1241 isolated from a soil sample collected in China by the present inventors. Taxonomical properties of the strain KP-1241 is illustrated hereinbelow.

### 1. Morphological properties:

The vegetative mycelia grow abundantly on both synthetic and complex agar media. and do not show fragmentation into coccoid forms or bacillary elements. The aerial mycelia grow abundantly on inorganic salts-starch agar and glycerolasparagine agar to show grayish color. The mature sporophores form spiral spore chains and have more than 20 spores per chain. The spores are oval in shape, 1.0 µm × 0.7 µm in size and have a spiny surface. Whirls, sclerotic granules, sporangia and flagellated spores are not observed.

### 2. Cultural characteristics:

The cultural characteristics are shown in Table 1. To investigate the cultural characteristics and physiological properties of the strain, the International Streptomyces Project (ISP) media recommended by Shirling and Gottlieb [Int. J. Syst. Bacteriol., 16 : 313-340. (1966) ] was used. Color Harmony Manual, 4th Ed., 1958 (Container Corporation of America, Chicago) was used for color names and hue numbers. Cultures were observed after incubation at 27 °C for two weeks, if not specified.

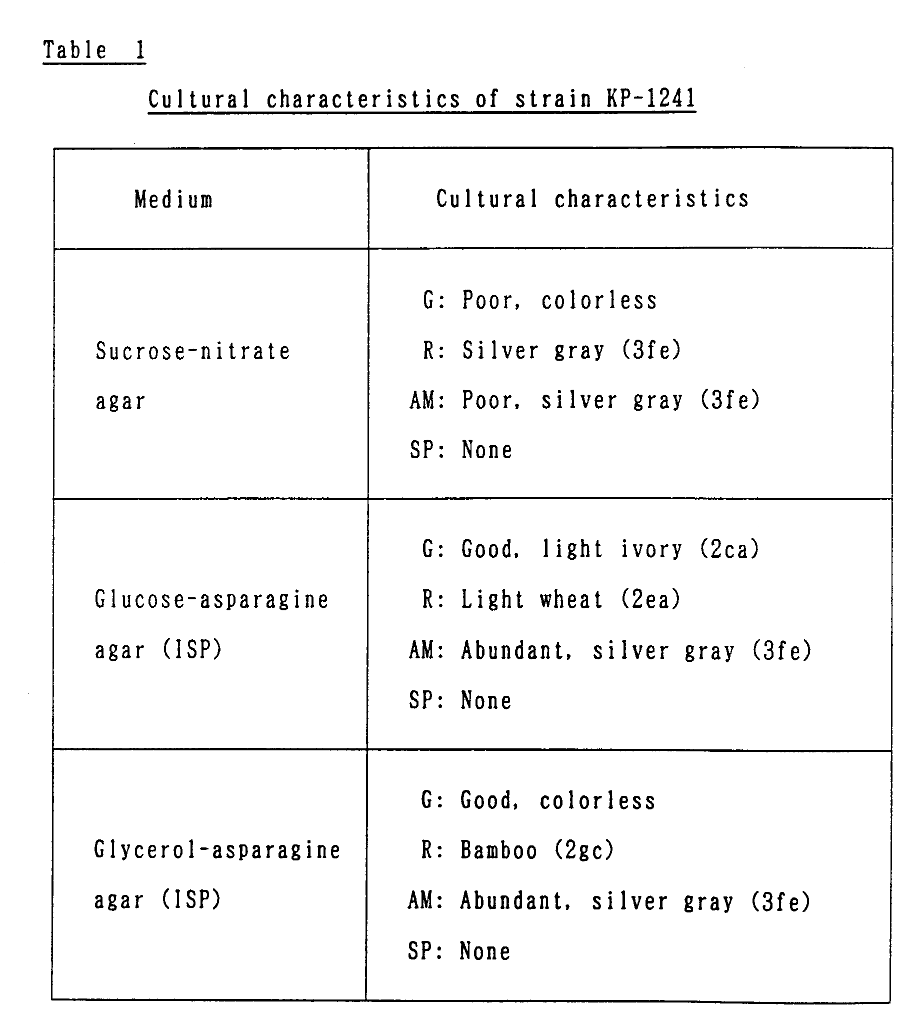

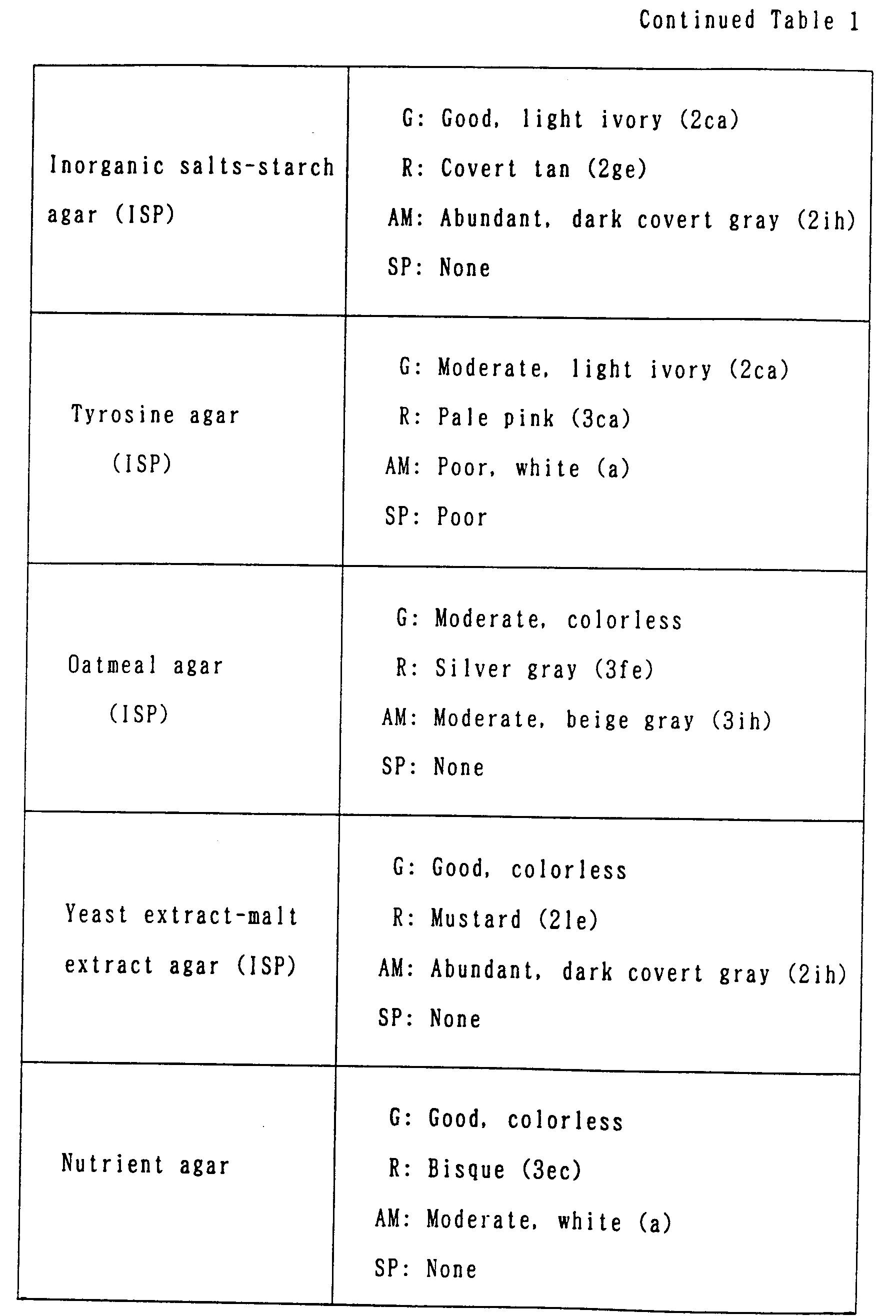

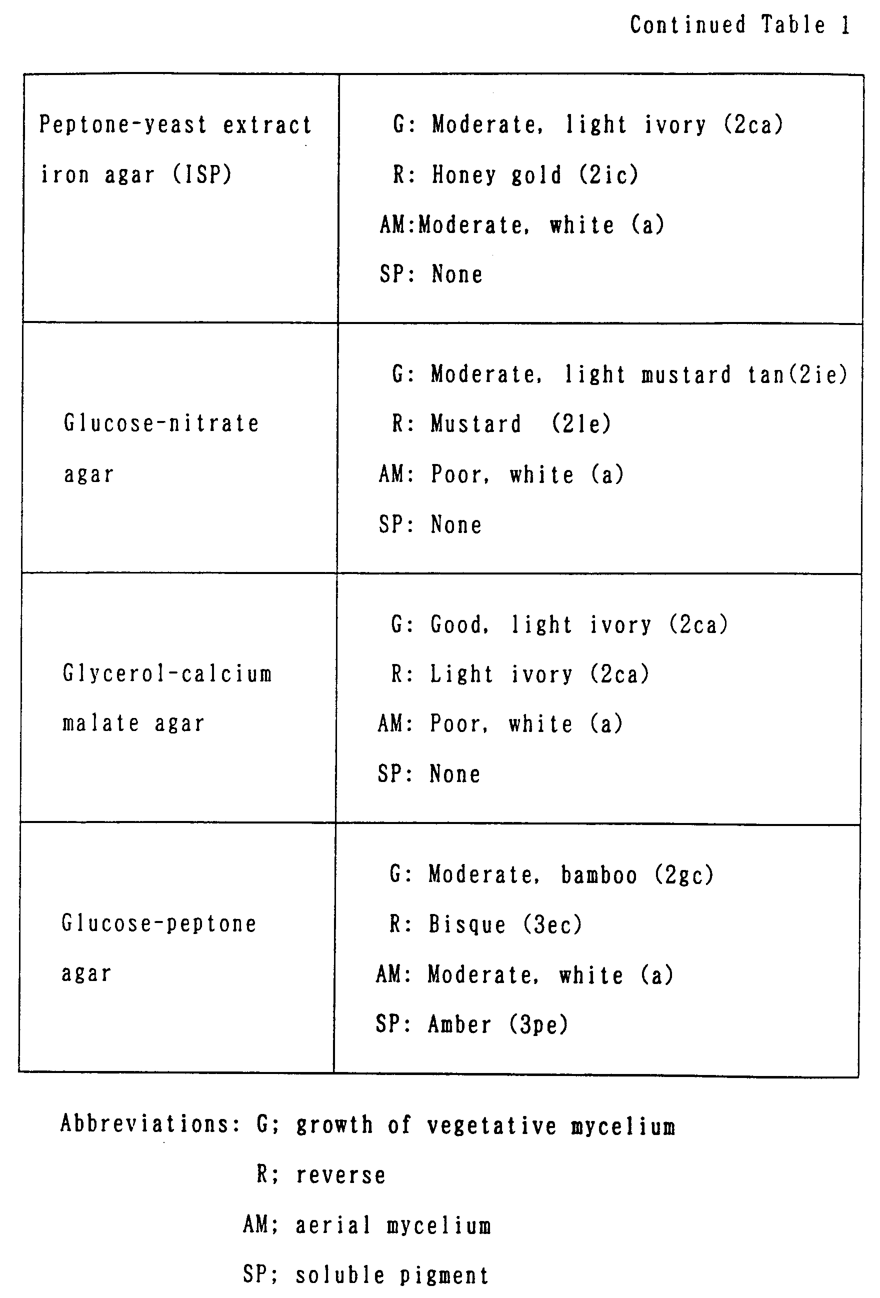

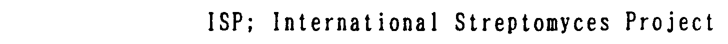

### 3. Physiological properties:

| | | (+ : Active, - : inactive) |
|---|---|---|
| (1) | Melanin formation | |
| | (a) Tyrosine agar | + |
| | (b) Peptone-yeast extract iron agar | + |
| | (c) Glucose-peptone-gelatin medium (21-23°C) | - |
| | (d) Tryptone-yeast liq. | - |
| (2) | Tyrosinase reaction | + |
| (3) | H₂S production | - |
| (4) | Nitrate reduction | + |
| (5) | Liquefaction of gelatin (21-23°C) | + |
| (6) | Hydrolysis of starch | + |
| (7) | Coagulation of milk (27°C) | - |
| (8) | Peptonization of milk (27°C) | + |
| (9) | Temperature range for growth | 12-34°C |
| | Optimum temperature range for growth | 24°C |
| (10) | Utilization of carbon sources | |
| | (Pridham and Gottlieb agar medium) | |
| | Utilized: D-glucose, D-mannitol, D-fructose, i-Inositol | |
| | Weakly utilized: L-arabinose, D-rhamnose | |
| | Not utilized: D-xylose, raffinose, melibiose, sucrose | |
| (11) | Cellulolytic activity | - |

### 4. Chemical composition:

The DAP (diaminopimelic acid)-isomer in the cell wall of strain KP-1241 is determined to be LL-type.

Taxonomical properties of the strain KP-1241 are illustratively summarized as follows. The DAP-isomer in the cell wall is LL-type. The aerial mycelia form spiral spore chains with smooth surface. In the cultural characteristics, the vegetative mycelia show white or gray on various media. Soluble pigment is slightly produced in tyrosine agar and glucose-peptone agar.

From the taxonomic properties described above, strain KP-1241 is considered to the white or gray series of the genus Streptomyces according to the classification by Pridham and Tresner (Bergey's Manual of Determinative Bacteriology, Vol. 8, pp. 748-829. 1974). The strain is referred to genus Streptomyces and is designated as Streptomyces sp. KP-1241. The strain was deposited in National Institute of Bioscience and Human-Technology. Agency of Industrial Science and Technology, Japan, under the name Streptomyces sp. KP-1241 and the accession No. is FERM BP-4171.

In the present invention, the above strain is merely illustrative, and the above strain, its mutant and the cystamidin A producing microorganism belonging to genus Streptomyces can be naturally used. The calpain inhibitor, cystamidin A of the present invention can be produced by inoculating and culturing aerobically the above strain, for example, in a nutrient medium suitable for cystamidin A production. Nutrient source such as nutrient meidum for Streptomyces can be used.

Commercially available nitrogen sources such as peptone, meat extract, corn steep liquor, cotton seed meal, peanut meal, soybean meal, yeast extract, NZ-amine, casein hydrolysate, sodium nitrate, ammonium nitrate and ammonium sulfate, and carbon sources such as glycerin, starch, glucose, galactose, lactose and mannose can be used. Further, carbon sources such as fatty substance, and inorganic salts such as sodium chloride, phosphate salts, calcium carbonate and magnesium sulfate can also be used.

The other essential trace metallic salt and anti-foam agent such as animal, vegetable or mineral oil can be added to the medium if required. These nutrient sources and other additives useful for production of cystamidin A can be used and that is to say any cultural material for Streptomyces can preferably be used in the present invention. Liquid culture such as submerged aeration culture is preferable for mass production of cystamidin A. Temperature for culturing the microorganisms can be adjusted to grow the strain within a range of suitable production of cystamidin A. Culturing condition can be selected and controlled for production of cystamidin A in which depending upon the nature of the above production microorganisms.

Cystamidin A is mainly produced in a cultured broth. A crude substance of cystamidin A can be isolataed by extracting the cultured filtrate with water immiscible organic solvent such as butanol, or by eluting the absorbed substance in an absorption resin with water-containing organic solvent. In addition to the above extraction procedure, the isolation method applied for low molecular weight substance such as absorption chromatography, gel-filtration chromatography, preparative thin layer chromatography, counter current partition chromatography and high performace liquid chromatography, and preferable combination and repeating operation thereof, can be applied for isolating and obtaining pure cystamidin A.

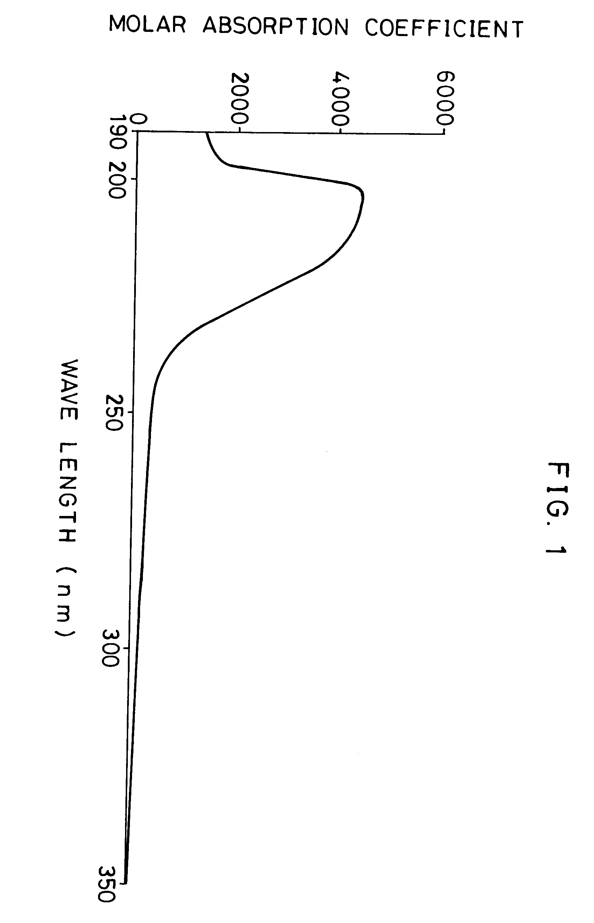

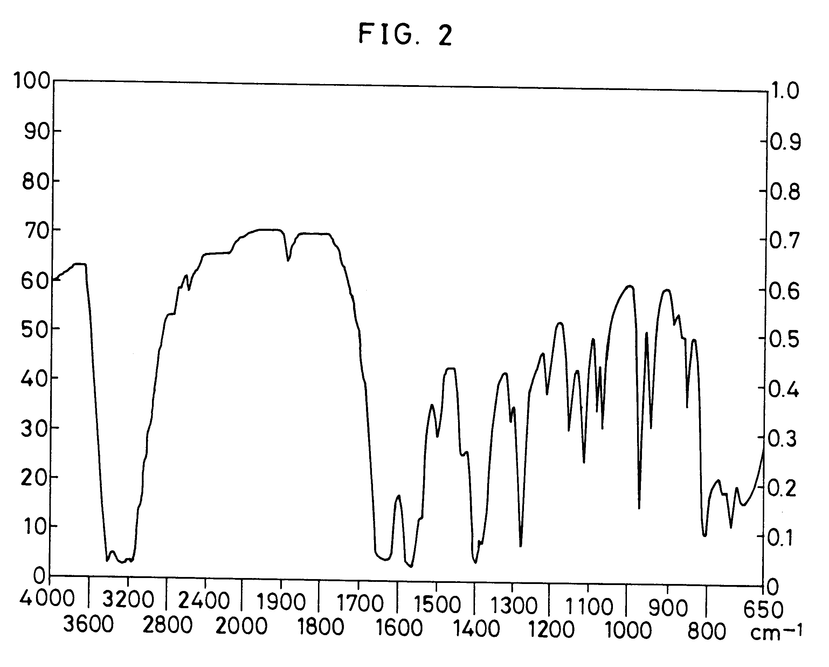

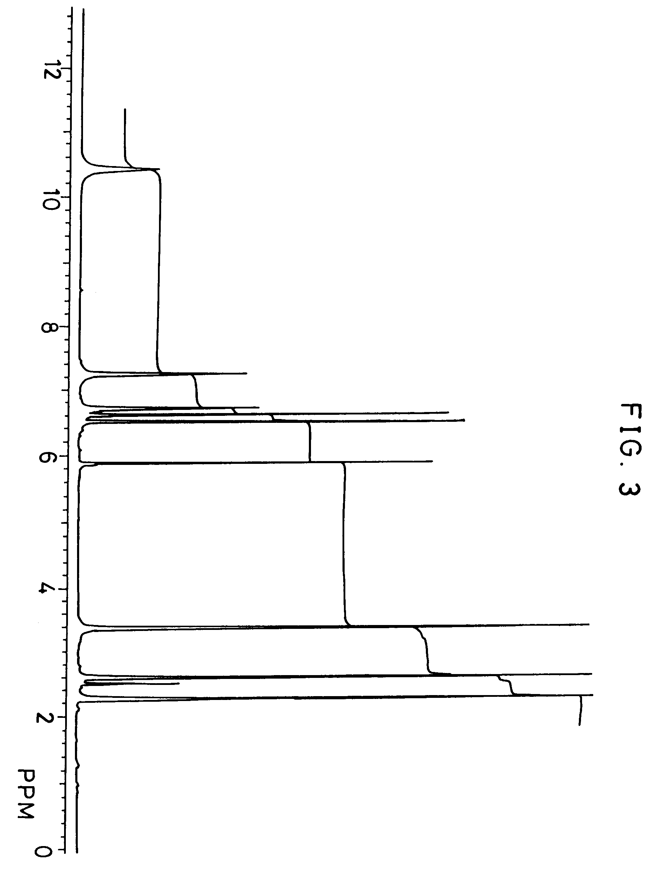

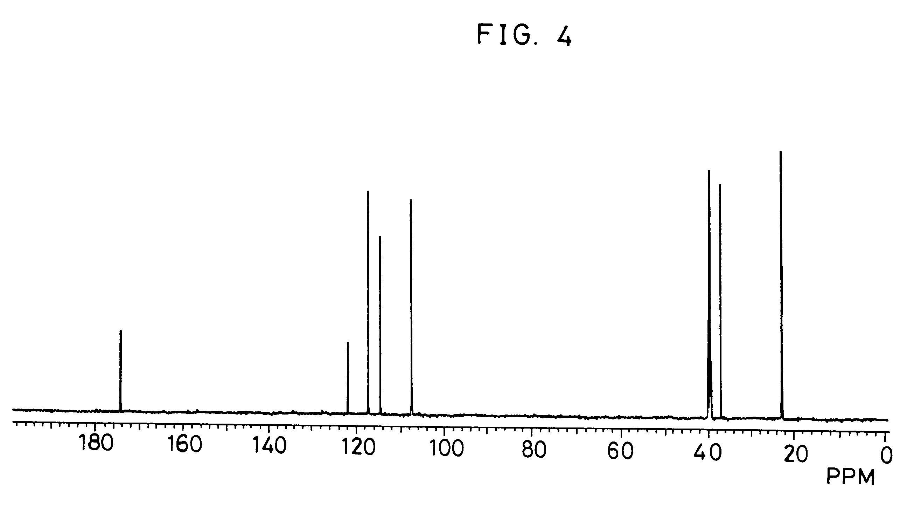

Physico-chemical properties of cystamidin A are shown in the followings.
(1) Nature : white powder
(2) Molecular weight : 138.0793 (by mass spectrometric analysis)
(3) Molecular formula: C₇H₁₀N₂O
(4) Melting point : 146 - 148 °C
(5) Ultraviolet absorption maximum (in methanol) : (Fig.1)
   203 nm ( ε = 4400), 213 nm (shoulder, ε = 4100), 260 - 280 nm (shoulder, ε = 300)
(6) Infrared absorption maximum (KBr tablet) : (Fig. 2)
   3400, 3250, 3150, 1640, 1570, 1400, 1280, 1110, 970, 805, 740 cm ⁻¹
(7) ¹H-NMR spectrum (in DMSO-d₆) : (Fig. 3)(ppm)
   10.40 br. s (1H), 7.24 br. s (1H), 6.69 br. s(1H), 6.60 dd (1H), 6.50 dd (1H), 5.86 dd (1H), 2.59 t (2H), 2.25 t (2H) ppm
   (s; singlet, d; doublet, t; triplet, br; broad)
(8) ¹³C-NMR spectrum (in DMSO-d₆) : (Fig. 4)(ppm)
   174.3 s, 121.8 s, 117.3 d, 114.6 d, 107.5 d, 37.0 t, 22.8 t
(9) Solubility in solvent : soluble in water, methanol, acetone, chloroform and benzene
(10) Color reaction : positive for ninhydrin, Ehrlich and Rydon-Smith reagents

From the above physico-chemical properties and the spectra data, the structure of cystamidin A was elucidated as shown in the formula [I] hereinbefore.

As illustrated in details on the physico-chemical properties of cystamidin A, the substance has never been known and reported and is a novel compound.

Biological properties of cystamidin A are illustrated as follows.

Inhibitory activities of cystamidin A against calpain using casein as substrate according to the method by Saito et al. [Agr. Biol. Chem., 51 : 361 (1987) ] , IC₅₀ (50 % inhibition) is 0.20 µg/ml.

Cystamidin A shows no antimicrobial activities at 100 µg/disk (paper disk method) against various kinds of bacteria, yeast and fungi. IC₅₀ value against B-16 melanoma cells in vitro were more than 25 µg/ml. The LD₅₀ (ip) of cystamidin A in mice are >200 mg/kg.

### Effect of the Invention:

As explained hereinaboves, the novel compound cystamidin A is a low molecular weight substance and shows strong inhibitory action on calpain, therefore it can be used not only for reagent but also for pharmaceuticals.

### Examples:

The embodiments of the present invention will be illustratively explained in the following examples, but are not construed as limiting.

### Example 1

A loopful mycelia of a stock culture of Streptomyces sp. KP-1241 FERM BP-4171 was transferred into 14 flasks consisting of a 500-ml Erlenmeyer flask containing 100 ml of a medium consisting of glucose 0.1%. soluble starch (Kanto Chem. Co.) 2.4%. peptone (Kyokuto Pharmaceutical Industrial Co.) 0.3%, meat extract (Kyokuto Pharmaceutical Industrial Co.) 0.3%, yeast extract (Oriental Yeast Co.) 0.5% and CaCO₃ 0.4% (pH 7.0 before sterilization), and then incubated on a rotary shaker at 27 °C for 3 days to give a seed culture. The seed culture (1.4 lit.) was inoculated into a tank fermenter contaning 70 liters of a medium consisting of glycerol 2,0%, soybean meal 2.0% and NaCl 0.3% (pH 7.0 before sterilization), and incubated with agitation at 200 rpm and aeration at the rate of 35 lit./min. at 27°C for 4 days.

The cultured broth was centrifuged and the supernatant was charged on a column of Diaion HP2O (5 lit., Mitsubishi Chem. Ind. Ltd.) and eluted with 20% methanol. Eluate (25 lit.) containing calpain inhibitory acitvity was extracted twice with ethyl acetate (18 lit.). The extract was concentrated in vacuo to obtain brownish oil I (55g). The oil was dissolved in water (1.5 lit.), put on a reversed phase sillica gel column (1000 ml. YMC * GEL ODS-AQ 120-S50. YMC Co.) packed with 50% methanol, and developed with 20% methanol.

Active fractions were collected and concentrated in vacuo to obtain brownish oil II (7.2 g). The oil was dissolved in a small volume of methanol, and the solution was mixed with silica gel powder (50 g, Merck Art. 7734), then methanol was removed from the mixture under reduced pressure. the dryed silica gel was put on a column of silica gel (210 g, Merck Art 7734) and eluted with a mixture of chloroform and methanol (30 : 1). Active fraction was collected and evaporated under reduced pressure to give brownish oil III (439 mg).

The oil was dissolved in a small volume of methanol and applied on HPLC (Capcell Pak C18, SG120, φ 20 × 250 mm, Shiseido Co.) separately with 12 times, with 5% acetonitrile at a flow rate of 8 ml/min. by checking and detecting with absorption at 205 nm. Peak showing calpain inhibitory activity, eluted at 17 min. was collected. The extract was concentrated under reduced pressure to obtain white powder of cystamidin A (370 mg).

## Claims

1. A compound of formula: 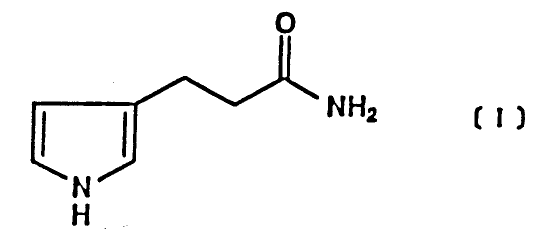 or a pharmaceutically or veterinarilly acceptable salt thereof.

2. A process for the production of a compound of formula (I), or a pharmaceutically or veterinarilly acceptable salt thereof, according to claim 1 which comprises culturing a microorganism belonging to the genus Streptomyces which is capable of producing the compound of formula (I), in a nutrient medium, isolating the compound of formula (I) from the cultured mass and, if desired, salifying the compound of formula (I) thus obtained.

3. A process according to claim 2 wherein the microorganism is Streptomyces sp. KP-1241 (FERM BP-4171) or a mutant thereof which is capable of producing a compound of formula (I) as depicted in claim 1.

4. A compound of formula (I), or a pharmaceutically or veterinarilly acceptable salt thereof, as depicted in claim 1 for use in a method of treatment of the human or animal body by therapy.

5. A compound of formula (I), or a pharmaceutically or veterinarilly acceptable salt thereof, as depicted in claim 1 for use as a calpain inhibitor in a method of treatment of the human or animal body by therapy.

6. Use of a compound of formula (I), or a pharmaceutically or veterinarilly acceptable salt thereof, as depicted in claim 1 in the manufacture of a medicament for use as a calpain inhibitor.

7. A pharmaceutical or veterinary composition which comprises a compound of formula (I), or a pharmaceutically or veterinarilly acceptable salt thereof, as depicted in claim 1 and a pharmaceutically or veterinarilly acceptable diluent or excipient.

8. Streptomyces sp. KP-1241 (FERM BP-4171) or a mutant thereof which is capable of producing a compound of formula (I) as depicted in claim 1.

9. A substantially pure culture of Streptomyces sp. KP-1241 (FERM BP-4171), or a mutant thereof which is capable of producing a compound of formula (I) as depicted in claim 1, comprising a source of assimilable carbon, a source of assimilable nitrogen and inorganic salts.

10. A compound which has the following physico-chemical properties:
(1) Nature : white powder;
(2) Molecular weight : 138.0793 (by mass spectrometric analysis);
(3) Molecular formula: C₇H₁₀N₂O;
(4) Melting point: 146 - 148°C;
(5) Ultraviolet absorption maxima (in methanol):
203 nm (ε=4400), 213 nm (shoulder, ε=4100), 260 - 280 nm (shoulder, ε=300);
(6) Infrared absorption maxima (KBr tablet):
3400, 3250, 3150, 1640, 1570, 1400, 1280, 1110, 970, 805, 740 cm⁻¹;
(7) Solubility in solvents: soluble in water, methanol, acetone, chloroform and benzene; and
(8) Colour reaction: positive for ninhydrin, Ehrlich and Rydon-Smith reagents;
or a pharmaceutically or veterinarilly acceptable salt thereof.

11. A process for the production of a compound according to claim 10, or a pharmaceutically or veterinarilly acceptable salt thereof, which comprises culturing a microorganism belonging to the genus Streptomyces which is capable of producing said compound in a nutrient medium, isolating said compound from the cultured mass, and, if desired, salifying the compound thus obtained.
